# EUROPEAN PATENT APPLICATION

(11) **EP 4 473 898 A1**
(43) Date of publication of application: **11.12.2024**
(21) Application number: 24168917.3
(22) Date of filing: 08.04.2024
(51) Int. Cl.: A61B 5/00, G06F 3/00, A61B 5/0531, A61B 5/11, G06F 3/01, G06F 3/03

(54) **INPUT METHODS PERFORMED AT WEARABLE DEVICES, AND SYSTEMS AND METHODS OF USE THEREOF**

(30) Priority: 27.04.2023 US 202363498756 P; 25.03.2024 US 202418614984
(71) Applicant: Meta Platforms Technologies, LLC, Menlo Park, CA 94025 (US)
(72) Inventor: Kienzle, Wolf, Menlo Park (US); Busquets, Roger Boldu, Menlo Park (US); Waghmare, Anandghan, Menlo Park (US); Whitmire, Eric, Menlo Park (US); Ng, Shiu Sang, Menlo Park, 94025 (US); Levi, Andre, Menlo Park (US)
(74) Representative: Murgitroyd & Company

(57) **Abstract**

The disclosed apparatus may include a wearable optic ring that features input capabilities relative to a computing system. In various examples, the wearable optic ring may be designed to curve around a wearer's finger and can detect complex interactions between that finger and the wearer's thumb. Moreover, the optical ring may detect such complex interactions based on ultra low-resolution images captured by a miniature optical sensor mounted on the optical ring and bioimpedance measurements taken by one or more electrodes included on the optical ring. Various other implementations are also disclosed.

## Description

### SUMMARY

According to an aspect, there is provided an apparatus comprising:
a ring designed to curve around a finger of a wearer, wherein the ring comprises an external surface and an internal surface configured to come into contact with the finger of the wearer;
a miniature optical sensor mounted on the external surface of the ring and positioned to view along a length of the finger of the wearer; and
one or more electrodes mounted on the internal surface of the ring, wherein the one or more electrodes serve to measure bioimpedance in the finger of the wearer.

The apparatus may further comprise an inertial measurement unit that measures movement of the ring.

The inertial measurement unit may measure movement of the ring in at least nine axes.

The apparatus may further comprise an LED mounted in association with the miniature optical sensor.

The miniature optical sensor may captures images of the finger of the wearer illuminated by the LED.

The finger of the wearer may be an index finger.

The miniature optical sensor may capture ultra low-resolution images of a thumb finger of the wearer interacting with the index finger of the wearer.

According to another aspect, there is provided a method comprising:
integrating a ring designed to curve around a finger of a wearer with a miniature optical sensor,
wherein the ring comprises an external surface and an internal surface configured to come into contact with the finger of the wearer and
the miniature optical sensor is integrated into the external surface of the ring at a position that allows the miniature optical sensor to view along a length of the finger of the wearer; and
integrating one or more electrodes into the internal surface of the ring, wherein the one or more electrodes serve to measure bioimpedance in the finger of the wearer.

The method may further comprise integrating an inertial measurement unit into the ring that measures movement of the ring in at least nine axes.

The method may further comprise integrating an LED into the ring that illuminates images captured by the miniature optical sensor.

The method may further comprise integrating a controller that determines movement of the ring based on images captured by the miniature optical sensor and measurements made by the inertial measurement unit,
and further optionally wherein the controller further determines an interaction of a thumb of the wearer relative to the ring based on images captured by the miniature optical sensor and bioimpedance measurements made by the one or more electrodes.

The interaction of the thumb of the wearer relative to the ring may comprise:
a touch of the thumb with a distal end of the finger of the wearer relative to the ring,
a touch of the thumb with a proximal end of the finger of the wearer relative to the ring,
a touch-and-slide movement of the thumb along the finger of the wearer away from the ring, or
a touch-and-slide movement of the thumb along the finger of the wearer toward the ring.

The controller may detect combination movement including movement of the ring and the interaction of the thumb of the wearer relative to the ring.

According to a further aspect, there is provided a method comprising:
receiving one or more ultra low-resolution images captured by a miniature optical sensor integrated into an external surface of a ring worn on a finger of a wearer;
receiving one or more inertial measurements from an inertial measurement unit integrated into the ring;
receiving one or more bioimpedance measurements captured by one or more electrodes integrated into an internal surface of the ring; and
determining a combination input gesture performed by the wearer based on the one or more ultra low-resolution images, the one or more inertial measurements, and the one or more bioimpedance measurements.

The combination input gesture may comprise:
a pinch-rotate-release input gesture that mimics the wearer turning a knob, or
a thumb-slide/wrist-flick input gesture.

The features and advantages described in the specification are not necessarily all inclusive and, in particular, certain additional features and advantages will be apparent to one of ordinary skill in the art in view of the drawings, specification, and claims. Moreover, it should be noted that the language used in the specification has been principally selected for readability and instructional purposes.

It will be appreciated that any features described herein as being suitable for incorporation into one or more aspects or embodiments of the present disclosure are intended to be generalizable across any and all aspects and embodiments of the present disclosure. Other aspects of the present disclosure can be understood by those skilled in the art in light of the description, the claims, and the drawings of the present disclosure. The foregoing general description and the following detailed description are exemplary and explanatory only and are not restrictive of the claims.

Having summarized the above example aspects, a brief description of the drawings will now be presented.

### BRIEF DESCRIPTION OF THE DRAWINGS

The accompanying drawings illustrate a number of exemplary implementations and are a part of the specification. Together with the following description, these drawings demonstrate and explain various principles of the present disclosure.
FIG. 1A illustrates an example optical ring including a miniature optical sensor in accordance with one or more implementations.
FIG. 1B illustrates a perspective view of the example haptic ring of FIG. 1A further showing one or more electrodes and an inertial measuring unit in accordance with one or more implementations.
FIGS. 2A and 2B illustrate the optical ring of FIGS. 1A and 1B detecting a pinch input gesture between an index finger and thumb of a wearer in accordance with one or more implementations.
FIGS. 3A and 3B illustrate the optical ring detecting movement of the hand of the wearer in multiple directions in accordance with one or more implementations.
FIGS. 4A and 4B illustrate the optical ring detecting a stateful touch-and-slide interaction between the thumb and the index finger of the wearer in accordance with one or more implementations.
FIGS. 5A and 5B illustrate the optical ring detecting another type of stateful pinch interaction between the thumb and the index finger of the hand of the wearer in accordance with one or more implementations.
FIGS. 6A and 6B illustrate the optical ring detecting a complex and stateful thumb-touch/wrist-flick input gesture in accordance with one or more implementations.
FIGS. 7A and 7B illustrate the optical ring detecting a scrubbing, side-to-side stateful input gesture in accordance with one or more implementations.
FIGS. 8A and 8B illustrate the optical ring detecting a stateful pinch-rotate-release input gesture that mimics the wearer turning a knob in accordance with one or more implementations.
FIGS. 9A and 9B illustrate the optical ring detecting a stateful writing input gesture by the hand of the wearer in accordance with one or more implementations.
FIG. 10 illustrates a detailed diagram of an optical ring control system for use in connection with the optical ring illustrated in FIGS. 1A-9B in accordance with one or more implementations.
FIG. 11 is an illustration of example augmented-reality glasses that may be used in connection with embodiments of this disclosure.
FIG. 12 is an illustration of an example virtual-reality headset that may be used in connection with embodiments of this disclosure.
FIG. 13 is an illustration of example haptic devices that may be used in connection with embodiments of this disclosure.
FIG. 14 is an illustration of an example virtual-reality environment according to embodiments of this disclosure.
FIG. 15 is an illustration of an example augmented-reality environment according to embodiments of this disclosure.

Throughout the drawings, identical reference characters and descriptions indicate similar, but not necessarily identical, elements. While the exemplary implementations described herein are susceptible to various modifications and alternative forms, specific implementations have been shown by way of example in the drawings and will be described in detail herein. However, the exemplary implementations described herein are not intended to be limited to the particular forms disclosed. Rather, the present disclosure covers all modifications, equivalents, and alternatives falling within the scope of the appended claims.

### DETAILED DESCRIPTION OF EXEMPLARY IMPLEMENTATIONS

Mixed-reality (XR) wearables, like augmented reality (AR) and virtual reality (VR) headsets, are becoming more useful and versatile. It follows that the XR input devices are needed to maximize the potential of these wearable devices. Existing XR input devices can often require wearers to make large and apparent hand motions to make selections, scroll content, interact with virtual objects and so forth. This can be distracting and tiring for the wearer. Moreover, existing XR input devices often require wearer-specific training data for underlying inference models to work effectively. Furthermore, existing XR input devices frequently lack the capability to detect complex "stateful" input gestures such as interactions that include both hand movement and finger interactions (e.g., such as pinches, touches, slides).

To address these issues, the present disclosure is generally directed to an optical ring that enables complex "stateful" input gestures. For example, one or more of the implementations described herein include an optical ring that may be worn on an index finger of a wearer. In at least one implementation. The optical ring can include a miniature optical sensor (e.g., an RGB camera) to capture actions of both the thumb and index finger of the wearer. Because of the proximity of the thumb and index finger, the disclosed optical ring can detect subtle interactions that include small, nuanced movements of those fingers. Moreover, in most implementations, the optical ring further includes one or more electrodes that can measure bioimpedance associated with the wearer's fingers. As such, the disclosed optical ring can utilize images captured by the miniature optical sensor and bioimpedance measurements taken by the one or more electrodes to detect complex but nuanced input gestures that include combinations of hand movements and finger interactions.

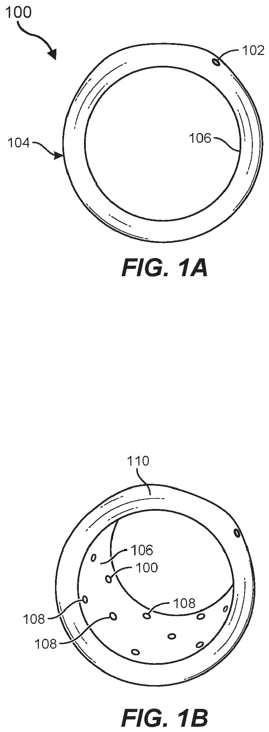

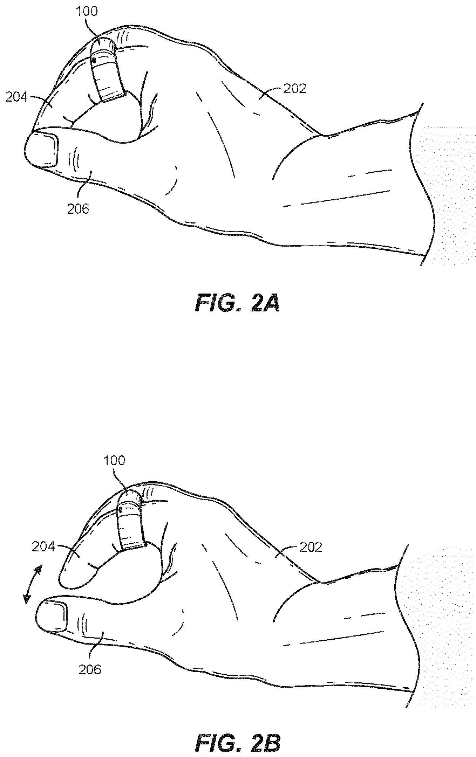

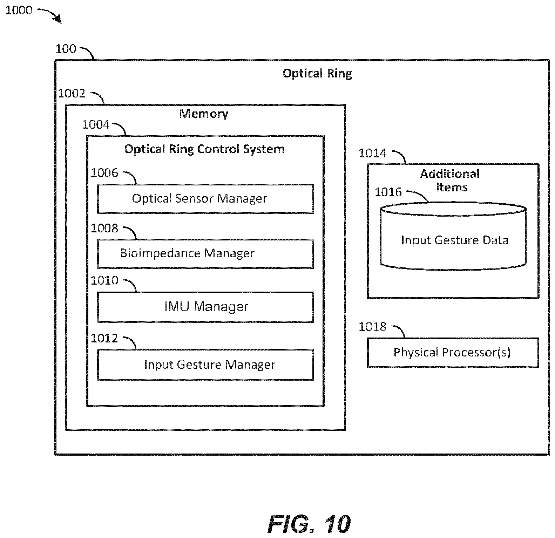

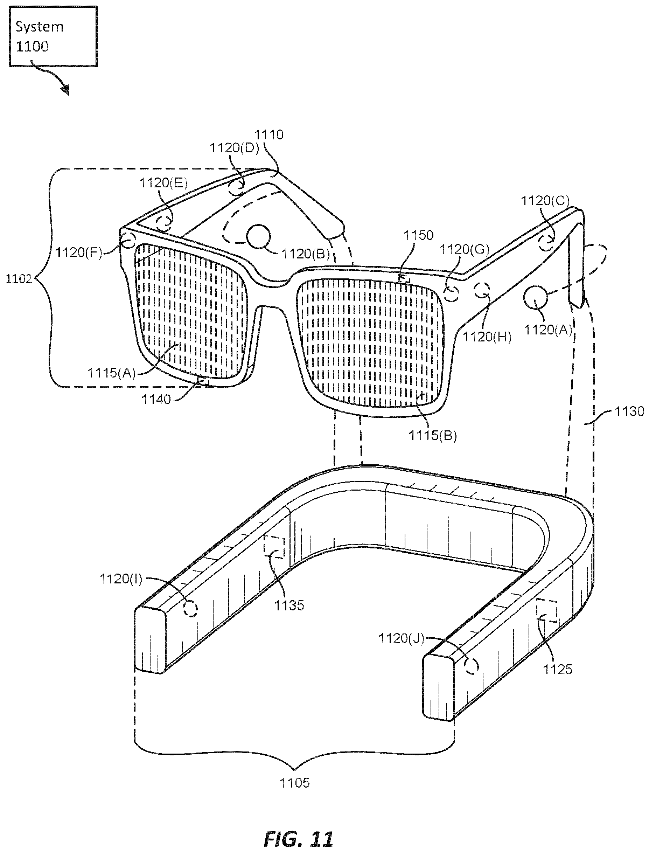

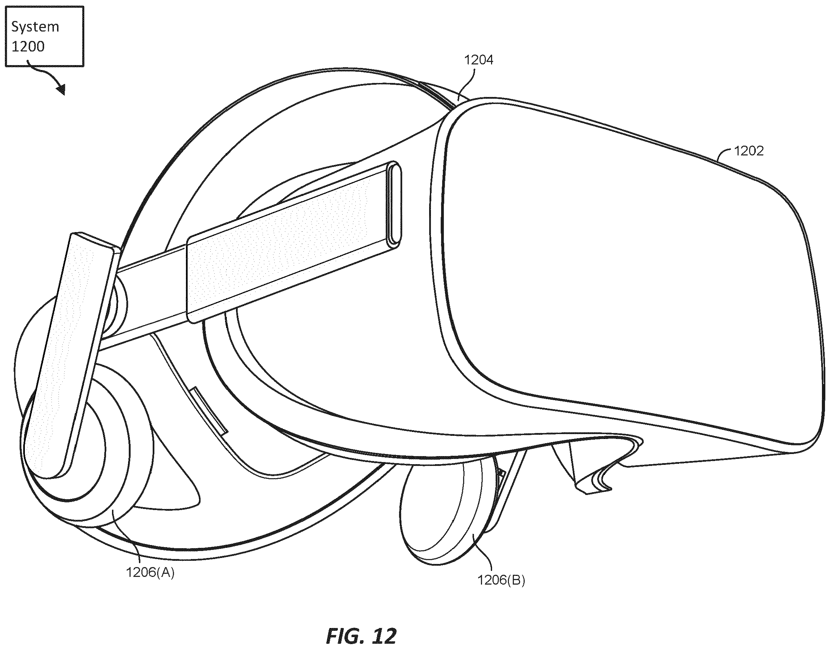

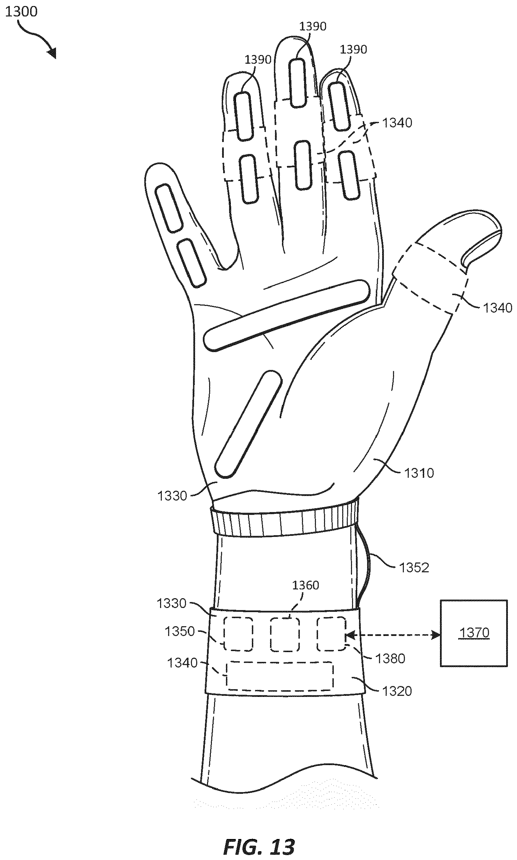

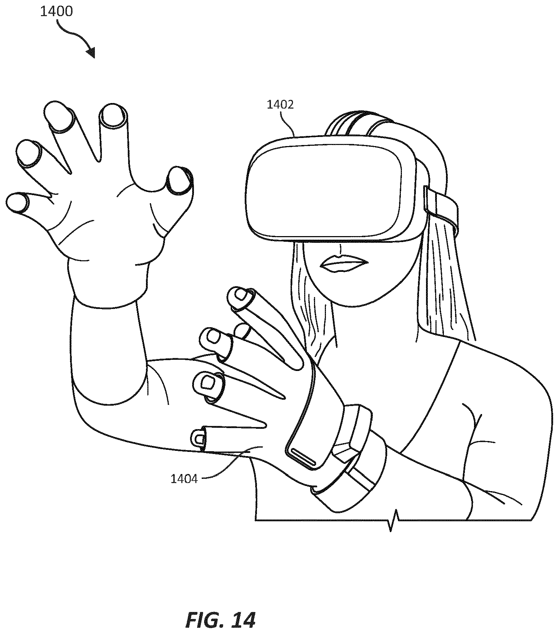

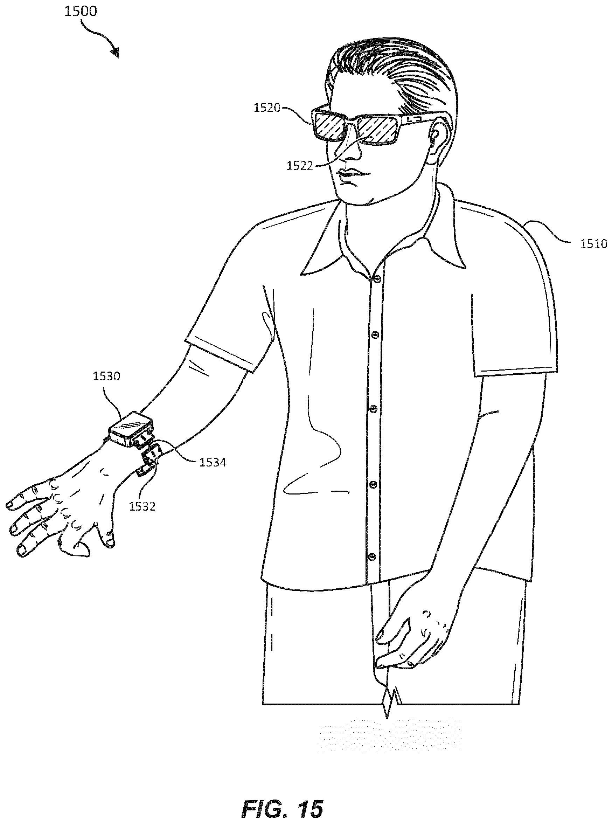

The following will provide, with reference to the FIGS. 1A-15, detailed descriptions of an optical ring. For example, FIGS. 1A and 1B illustrate views of the disclosed optical ring. FIGS. 2A-9B illustrate the disclosed optical ring detecting a wide range of complex, nuanced and stateful input gestures when worn by a wearer. FIG. 10 illustrates additional detail associated with an optical ring control system that operates in connection with the disclosed optical ring. FIG. 11 illustrates example augmented-reality glasses while FIG. 12 illustrates an example virtual-reality headset. FIG. 13 illustrates example haptic devices and FIG. 14 illustrates an example virtual-reality environment. Finally, FIG. 15 illustrates an example augmented-reality environment.

As just mentioned, FIGS. 1A and 1B illustrate an optical ring 100 that detects complex, "stateful" input gestures that include combinations of nuanced hand movements and finger interactions. For example, as shown in a side-view illustrated in FIG. 1A, the optical ring 100 is designed to curve around a finger of a wearer. In some implementations, as shown in FIG. 1A, the optical ring 100 can be closed so as to completely encircle the wearer's finger. In additional implementations, the optical ring 100 can be open so as to only partially encircle the wearer's finger.

As further shown in FIG. 1A, the optical ring 100 can include a miniature optical sensor 102. In one or more implementations, the optical ring 100 can include the miniature optical sensor 102 mounted onto an external surface 104 of the optical ring 100. Furthermore, the miniature optical sensor 102 can be mounted onto the external surface 104 of the optical ring 100 in a position that allows the miniature optical sensor 102 to view down a length of the finger of the wearer (e.g., toward the fingertip) to enable recognition of thumb-to-index finger interactions. In additional implementations, the optical ring 100 may include additional miniature optical sensors in the same or different positions on the optical ring 100. As will be discussed in greater detail below with reference to FIG. 10, the miniature optical sensor 102 can include an RGB camera that captures ultra low-resolution images.

FIG. 1B shows a perspective view of the optical ring 100. As shown in FIG. 1B, the optical ring 100 further includes one or more electrodes 108. In one or more implementations, the one or more electrodes 108 can be mounted on an internal surface 106 of the optical ring 100. For example, the one or more electrodes 108 can be positioned on the internal surface 106 of the optical ring 100 so as to come into contact with the skin of the wearer's finger. In at least one implementation, the one or more electrodes 108 serve to measure bioimpedance in the wearer's finger.

Additionally, in one or more implementations, the optical ring 100 can also include an inertial measurement unit (IMU) 110. For example, the IMU 110 can measure movement of the optical ring 100 in space. In at least one implementation, the IMU 110 can measure movement of the optical ring 100 in at least nine axes. To illustrate, the IMU 110 can measure up and down movement of the optical ring 100, back and forth movement of the optical ring 100, side to side movement of the optical ring 100, and diagonal movement of the optical ring 100.

Based on inputs detected by the miniature optical sensor 102, the one or more electrodes 108, and the IMU 110, the optical ring 100 can detect complex and "stateful" input gestures made by a wearer's hand and/or fingers. FIGS. 2A-9B illustrate additional detail with regard to such complex and "stateful" input gestures that can be detected by the optical ring 100. For example, FIGS. 2A and 2B illustrate the optical ring 100 detecting a pinch input gesture between an index finger 204 and thumb 206 of a hand 202 of the wearer. In one or more implementations, the optical ring 100 detects the index finger 204 and the thumb 206 coming into contact, as shown in FIG. 2A, based on images captured by the miniature optical sensor 102 and measurements taken by the one or more electrodes 108-in connection with measurements taken by the IMU 110. Similarly, as shown in FIG. 2B, the optical ring 100 detects separation of the index finger 204 and the thumb 206 (e.g., along the arrow shown) based on updates to the same inputs. In one or more implementations, the optical ring 100 detects a minute separation of the index finger 204 and the thumb 206 such that the wearer need not open the hand 202 more than a few millimeters.

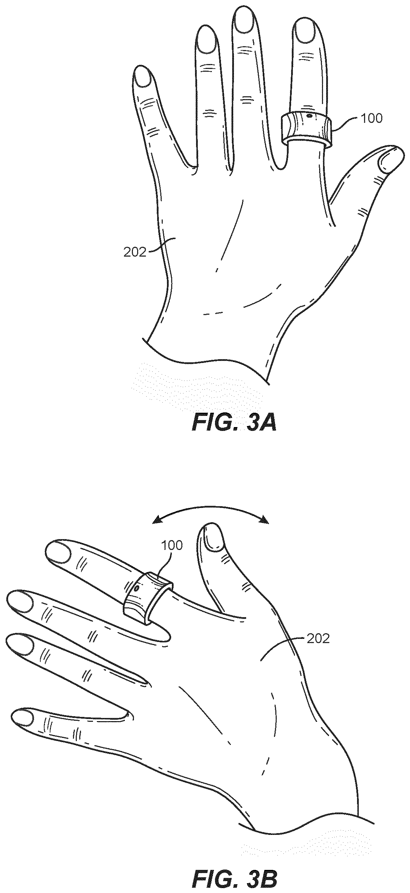

FIGS. 3A-3B illustrate the optical ring 100 detecting movement of the hand 202 of the wearer in multiple directions. For example, as shown in FIGS. 3A and 3B, the optical ring 100 can detect lateral rotation of the hand 202 of the wearer (e.g., a side-to-side movement along the arrow shown in FIG. 3B) based on measurements taken by the IMU 110. Similarly, the optical ring 100 can detect flexion of the wrist of the hand 202 or the arm of the wearer (e.g., an up-and-down movement of the hand 202) based on the same measurements. In some implementations, the optical ring 100 further refines the detection of these movements based on images captured by the miniature optical sensor 102.

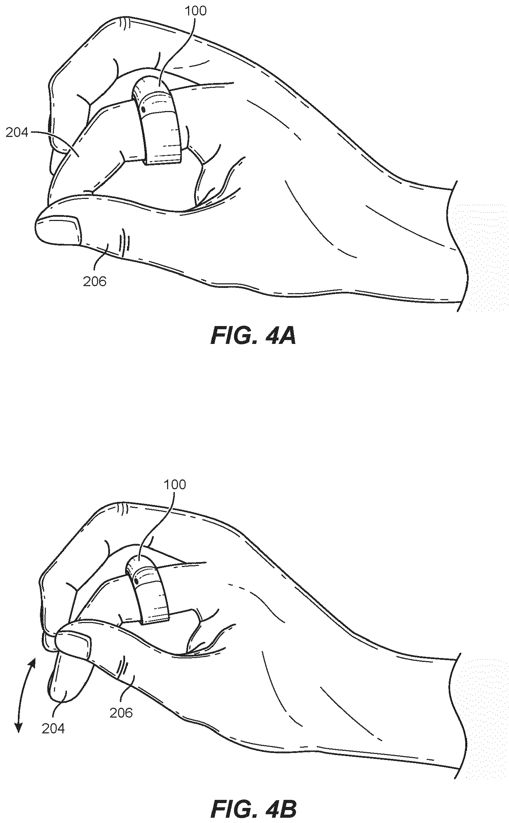

FIGS. 4A and 4B illustrate the optical ring 100 detecting a stateful touch-and-slide interaction between the thumb 206 and the index finger 204 of the wearer. For example, as shown in FIG. 4A, the optical ring 100 can detect the thumb 206 of the wearer coming into contact with the distal end of the index finger 204. The optical ring 100 can further detect the thumb 206 sliding along the index finger 204 toward the optical ring 100 and then away from the optical ring 100 (e.g., along the arrow shown in FIG. 4B). In one or more implementations, the optical ring 100 detects this touch-and-slide movement of the thumb 206 along the index finger 204 based on bioimpedance measurements taken by the one or more electrodes 108 and one or more images captured by the miniature optical sensor 102.

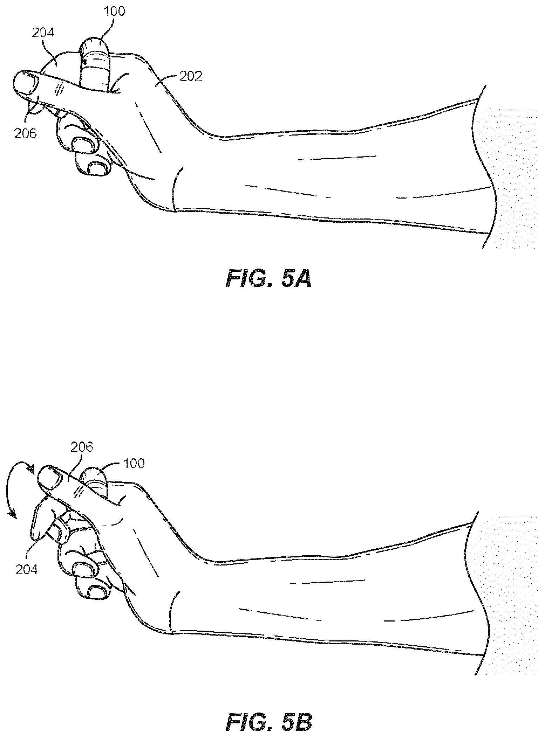

FIGS. 5A-5B further illustrate the optical ring 100 detecting anothertype of stateful pinch interaction between the thumb 206 and the index finger 204 of the hand 202 of the wearer. For example, the optical ring 100 can detect-and distinguish between-a touch of the thumb 206 with a distal end of the index finger 204, as shown in FIG. 5A, and a touch of the thumb 206 with a proximal end of the index finger 204, as shown in FIG. 5B (e.g., as the finger moves as indicated by the arrow shown in FIG. 5B). In one or more implementations, the optical ring 100 detects and distinguishes between touches of the thumb 206 with the index finger 204 at positions that are either near or far from the optical ring 100 based on bioimpedance measurements taken by the one or more electrodes 108 and one or more images captured by the miniature optical sensor 102.

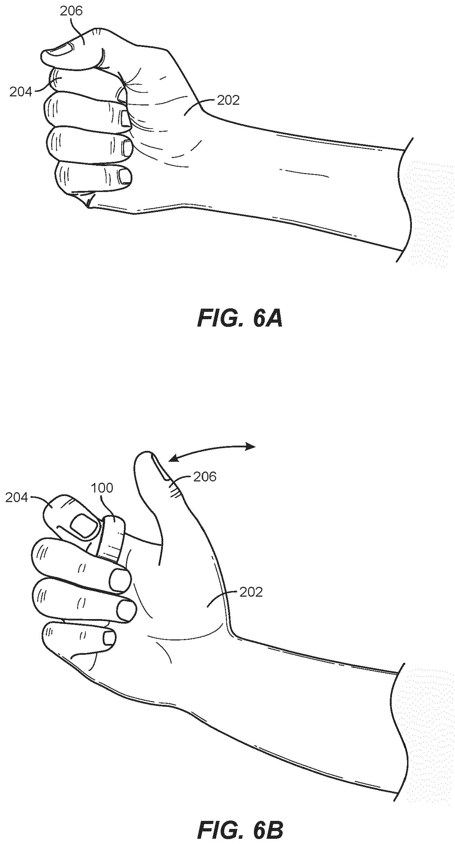

FIGS. 6A and 6B illustrate the optical ring 100 detecting a complex and stateful thumb-touch/wrist-flick input gesture. For example, as shown in FIG. 6A, the optical ring 100 can detect a starting position of the wearer's hand 202 including the thumb 206 in contact with the index finger 204 and the wrist of the hand 202 in a neutral position. The optical ring 100 can then detect an extended position of the wearer's hand 202 including the thumb 206 extending away from the index finger 204 (e.g., along the arrow shown in FIG. 6B) while the wrist of the hand 202 "flicking" or quickly shifting into a flexed position. As shown in FIG. 6B, the optical ring 100 can detect the wrist of the hand 202 flicking up. In additional implementations, the optical ring 100 can detect the wrist of the hand 202 flicking down or to the side. The optical ring 100 can detect this combination input gesture based on bioimpedance measurements taken by the one or more electrodes 108, movement measurements taken by the IMU 110, and one or more images captured by the miniature optical sensor 102.

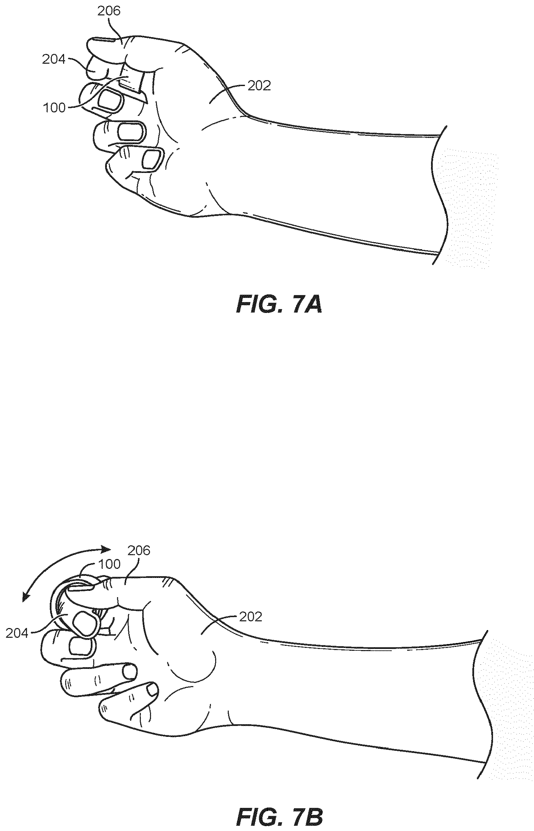

FIGS. 7A and 7B illustrate the optical ring 100 detecting a scrubbing, side-to-side stateful input gesture. For example, as shown in FIG. 7A, the optical ring 100 can detect the thumb 206 coming into contact with the index finger 204. Next, as shown in FIG. 7B, the optical ring 100 can further detect a sweeping, side-to-side motion of the hand 202 (e.g., along the arrow shown in FIG. 7B), while the thumb 206 remains in contact with the index finger 204. As with the input gesture illustrated in FIGS. 6A and 6B, the optical ring 100 can detect the side-to-side stateful input gesture illustrated in FIGS. 7A and 7B based on bioimpedance measurements taken by the one or more electrodes 108, movement measurements taken by the IMU 110, and one or more images captured by the miniature optical sensor 102.

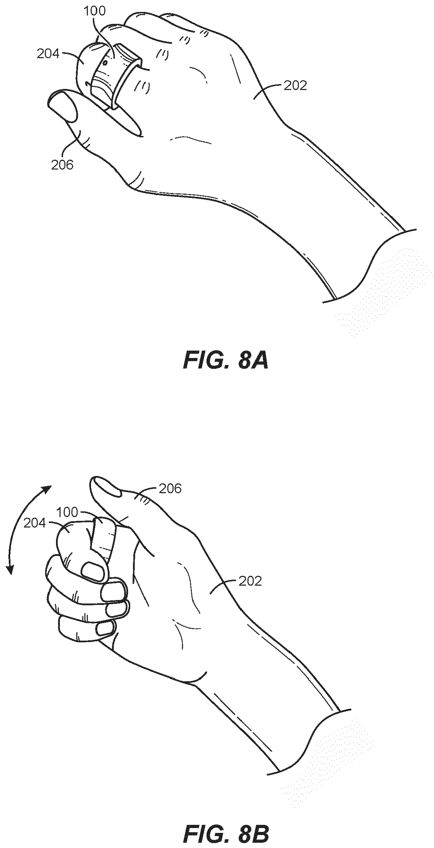

FIGS. 8A and 8B illustrate the optical ring 100 detecting a stateful pinch-rotate-release input gesture that mimics the wearer turning a knob. For example, as shown in FIG. 8A, the optical ring 100 can detect the thumb 206 coming into contact with the index finger 204 of the hand 202 of the wearer. The optical ring 100 can further detect the hand 202 rotating while the thumb 206 remains in contact with the index finger 204. Finally, the optical ring 100 can detect release of the thumb 206 following the rotation, as shown in FIG. 8B. As with the previous input gestures, the optical ring 100 can detect this pinch-rotate-release input gesture based on bioimpedance measurements taken by the one or more electrodes 108, movement measurements taken by the IMU 110, and one or more images captured by the miniature optical sensor 102.

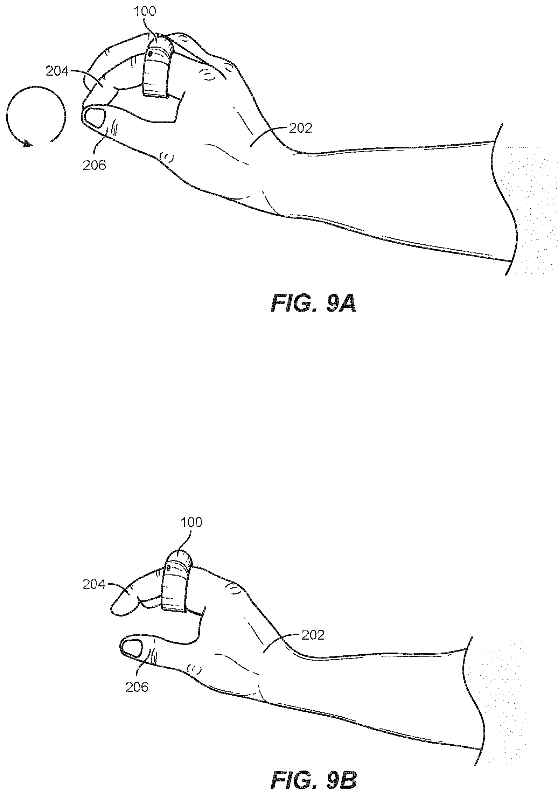

FIGS. 9A and 9B illustrate the optical ring 100 detecting a stateful writing input gesture by the hand 202 of the wearer. For example, as shown in FIG. 9A, the optical ring 100 can detect the thumb 206 pinching a distal end of the index finger 204. The optical ring 100 can further detect movement of the hand 202 while the thumb 206 is in contact with the index finger 204-as if the wearer were writing in mid-air along the arrow shown in FIG. 8A. Finally, the optical ring 100 can detect the thumb 206 release the index finger 204 to end the input gesture. As with the previous input gestures, the optical ring 100 can detect this pinch-rotate-release input gesture based on bioimpedance measurements taken by the one or more electrodes 108, movement measurements taken by the IMU 110, and one or more images captured by the miniature optical sensor 102.

As mentioned above, and as shown in FIG. 10, the optical ring 100 includes or is associated with a control system that takes inputs from one or more of the miniature optical sensor 102, the one or more electrodes 108, and the IMU 110 to determine and identify complex, "stateful" user input gestures. FIG. 10 is a block diagram 1000 of an optical ring control system 1004 operating within a memory 1002 of the optical ring 100 while performing these functions. As such, FIG. 10 provides additional detail with regard to these functions. For example, in one or more implementations as shown in FIG. 10, the optical ring control system 1004 can include an optical sensor manager 1006, a bioimpedance manager 1008, an IMU manager 1010, and an input gesture manager 1012. As further shown in FIG. 10, additional items 1014 stores and maintains input gesture data 1016. While the optical ring control system 1004 is illustrated in FIG. 10 within the optical ring 100, the optical ring control system 1004 may be located elsewhere in other implementations. For example, in an additional implementation, the optical ring control system 1004 may be located as part of an AR/VR system. In that implementation, the optical ring 100 may include one or more transmitters for transmitting input data captured by one or more of the miniature optical sensor 102, the one or more electrodes 108, and the IMU 110 to the optical ring control system 1004.

In certain implementations, the optical ring control system 1004 represents one or more software applications, modules, or programs that, when executed by a computing device, may cause the computing device to perform one or more tasks. For example, and as will be described in greater detail below, one or more of the optical sensor manager 1006, the bioimpedance manager 1008, the IMU manager 1010, and the input gesture manager 1012 may represent software stored and configured to run on one or more devices, such as the optical ring 100. One or more of the optical sensor manager 1006, the bioimpedance manager 1008, the IMU manager 1010, or the input gesture manager 1012 of the optical ring control system 1004 shown in FIG. 10 may also represent all or portions of one or more special purpose computers to perform one or more tasks.

As mentioned above, and as shown in FIG. 10, the optical ring control system 1004 includes the optical sensor manager 1006. In one or more implementations, the optical sensor manager 1006 handles tasks related to the miniature optical sensor 102. For example, in most implementations, the miniature optical sensor 102 includes a miniature RGB camera that captures actions of both the thumb 206 and index finger 204 of the wearer (or other finger if the wearer cannot use their index finger). In some implementations, the miniature optical sensor 102 can be a miniature, low resolution camera. Additionally, in some implementations, the optical ring 100 can further include an LED that enables the optical ring 100 to operate in low-light settings and perform computational operations such as background subtraction with greater efficiency.

In more detail, the optical sensor manager 1006 enables the miniature optical sensor 102 to be an ultra low-resolution camera, such as one that captures images at 5x5 pixels. In most implementations, this ultra low-resolution preserves wearer privacy while reducing computational needs. In one or more implementations, the optical sensor manager 1006 utilizes ultra low-resolution images captured by the miniature optical sensor 102 without any training data from the wearer by leveraging an underlying inference model that is based on user-independent heuristics and user-generalizable ML models.

To illustrate, the optical sensor manager 1006 can utilize an interaction recognition pipeline to glean input interaction information from ultra low-resolution images captured by the miniature optical sensor 102. In one or more implementations, the primary components of the interaction recognition pipeline can include finger segmentation to separate the fingers from the background in a capture image, input gating to remove false positives, and interaction recognition to identify user-performed interactions.

During finger segmentation, the optical sensor manager 1006 employs color-based segmentation to separate fingers from the backdrop in an output image from the miniature optical sensor 102. In one or more implementations, the optical ring 100 further includes a red-colored LED that illuminates the thumb 206 and the index finger 204, which may strongly light up due to their proximity to the LED, while surfaces further away from the LED do not illuminate as intensely. The optical sensor manager 1006 can use the difference in intensity of the red areas to perform a color-based background segmentation and leave only the fingers. Using active illumination for segmentation serves to enable robust segmentations with a lightweight thresholding approach as opposed to using more computationally demanding methods, such as edge detection or other ML-based techniques. As such, the optical sensor manager 1006 can help conserve the limited computing resources of the optical ring 100. Robust segmentation further enables the use of heuristic-based algorithms for interaction recognition.

In one or more implementations, the optical sensor manager 1006 can perform the color-based thresholding by first converting the raw RGB image captured by the miniature optical sensor 102 to HSV color space, followed by applying a specified threshold (lower bound H=0, S=179, V=82 to upper bound H=179, S=255, V=255). Since the LED powerfully illuminates the fingers and completely overlays them with red light, the wearer's skin tone has little impact on color thresholds. As such, the same threshold values apply to all wearers. After color segmentations, the optical sensor manager 1006 can transform the segmented image into a binary image for further processing.

During input gating, the optical sensor manager 1006 can employ a machine learning classifier to differentiate between instances when wearers do not intend to make a gesture and when they are actively performing or planning to make a gesture. This helps to filter out false positives. In one or more implementations, the optical sensor manager 1006 trains the classifier using positive examples where a wearer is performing or preparing to perform a gesture and negative examples where a user is handling an object or carrying out an action that is not a valid interaction with their hand. In at least one implementation, the optical sensor manager 1006 utilizes a random forest classifier with one hundred trees for this classification task. In one or more implementations, the input to the classifier is a flattened-out binary image after segmentation.

During interaction recognition, the optical sensor manager 1006 can recognize both stateful gestures and other types of gestures. In more detail, the optical sensor manager 1006 can recognize a stateful pinch that occurs when the index finger 204 and the thumb 206 make contact. For example, the optical sensor manager 1006 detects these types of pinches by first identifying fingers using a contour detection algorithm in the segmented binary image. Among all the contours found, the optical sensor manager 1006 can filter out those with an area less than 30% of the total image area, as they do not represent a finger. When no pinch is performed, the image consists of two distinct contours representing the two fingers. At the moment of contact, the two contours blend into a single outline depicting the fingers in touch, representing a pinch gesture. When a single contour is detected, the optical sensor manager 1006 can perform an additional check to ensure that the width of the bounding box of the detected contour is equal to the width of the frame. If the contour's width matches the frame's width, the optical sensor manager 1006 can recognize the gesture as a pinch.

Additionally, the optical sensor manager 1006 can recognize and distinguish other gestures (e.g., a left swipe, a right swipe, a long tap) by analyzing the motion and duration of the thumb's movement along the index finger 204 while the two fingers are in contact (e.g., pinching). The optical sensor manager 1006 can track the thumb's movement by monitoring the area of contour representing the fingers. As the thumb 206 moves closer to the optical ring 100, its appearance in the miniature optical sensor 102 becomes bigger; hence, the contour area increases, and vice versa. The optical sensor manager 1006 can calculate the duration of the contact as the time between the start and end of the pinch. During a stateful pinch, the optical sensor manager 1006 can recognize a gesture as a left or right swipe depending on the direction of the slide. Conversely, the optical sensor manager 1006 can recognize a gesture as a long tap when there is little or no motion of the thumb 206 on the index finger 204.

In some implementations, the optical sensor manager 1006 makes these determinations using a heuristic-based method that considers the direction of movement and the duration of the movement. For example, in one implementation, the optical sensor manager 1006 recognizes a left swipe gesture when, for a number of frames between 5 and 20 (e.g., 0.15-0.6 sec) the sum of the contour area of the first two frames is greater than that of the last two frames-and vice versa for a right swipe gesture. In an additional implementation, the optical sensor manager 1006 recognizes a long tap gesture by recording the contour area of each frame during a valid pinch. When the pinch ends, the optical sensor manager 1006 examines the length of the recorded frames. The optical sensor manager 1006 may reject a pinch as an accidental touch when the pinch length is less than five frames. If the number of frames exceeds 20 (0.6 sec at 30fps) and is less than 40 (1.2 sec at 30fps), the optical sensor manager 1006 can determine that the gesture is a long tap.

In one or more implementations, the optical sensor manager 1006 can also track continuous 1D gestures. For example, when the wearer performs a continuous input interaction, the optical sensor manager 1006 can begin to track the thumb's position over the index finger 204 when contact between the two fingers is sensed. The optical sensor manager 1006 further tracks the thumb's position by monitoring the contour area in the frame and calculating the delta as the difference between the thumb's initial location when a pinch was first detected and its current location. The delta can be positive or negative depending on the direction of motion. Since the delta depends on the initial touch position, which differs each time, the optical sensor manager 1006 tracks the relative movement of the thumb 206, not its absolute position. This helps the optical sensor manager 1006 generalize across varied finger sizes and shapes.

As mentioned above, and as shown in FIG. 10, the optical ring control system 1004 includes the bioimpedance manager 1008. In one or more implementations, the bioimpedance manager 1008 measures impedance of the biological tissues of a wearer of the optical ring 100. For example, the bioimpedance manager 1008 can measure bioimpedance by assessing the resistance and reactance of the wearer's fingers to an electrical current that is applied by the one or more electrodes 108. In at least one implementation, the bioimpedance manager 1008 causes the one or more electrodes 108 to pass a low-level electrical current through the wearer's body and then measures the resulting voltage.

In more detail, biological tissue typically has various electrical properties. For example, resistance (R) represents the hindrance of the flow of electrical current and is mainly associated with non-conductive components like cell membranes and connective tissues. Additionally, reactance (X) indicates the delay in the flow of electrical current caused by capacitive effect, often related to cell membranes. In response to causing a first subset of the one or more electrodes 108 to apply a small, harmless electrical current (e.g., at frequencies between 1 kHz and 1MHz), the bioimpedance manager 1008 can further cause a second subset of the one or more electrodes 108 to measure the resulting voltage. The bioimpedance manager 1008 can calculate the impedance (Z) of the wearer's tissue using Ohm's Law: Z = V/I, where Z is impedance, V is voltage, and I is current. In one or more implementations, the bioimpedance manager 1008 can determine that the wearer's index finger 204 and thumb 206 are touching or not touching based on changes to the calculated impedance (Z).

As mentioned above, and as shown in FIG. 10, the optical ring control system 1004 includes the IMU manager 1010. In one or more implementations, the IMU manager 1010 determines how the hand 202 of the wearer (e.g., while wearing the optical ring 100) moves based on measurements taken by the IMU 110 (e.g., the inertial measurement unit). In one or more implementations, the IMU 110 can include various components such as a gyroscope, an accelerometer, and sometimes a magnetometer. For example, the gyroscope can track and measure how the optical ring 100 is rotating or turning. Additionally, the accelerometer can detect changes in speed or direction associated with the optical ring 100. In one or more implementations, the magnetometer can detect directionality of the optical ring 100 based on a magnetic field (e.g., Earth's magnetic field). Based on all of these measurements, the IMU manager 1010 can determine how the optical ring 100 is moving during input gestures.

As mentioned above, and as shown in FIG. 10, the optical ring control system 1004 includes the input gesture manager 1012. In one or more implementations, the input gesture manager 1012 takes the inputs and determinations of the optical sensor manager 1006, the bioimpedance manager 1008, and the IMU manager 1010 to determine the input gesture being made by the wearer of the optical ring 100. For example, the input gesture manager 1012 can utilize heuristics, algorithms, machine learning models, and other techniques to make this determination. In at least one implementation, the input gesture manager 1012 can transmit the determined input gesture to a computing system (e.g., a VR system, an AR system) for further utilization. In additional implementations, the input gesture manager 1012 can further make interaction determinations based on other input devices mounted on the optical ring 100 such as a contact microphone.

As shown in FIG. 10, the optical ring 100 includes one or more physical processors, such as the physical processor(s) 1018. The physical processor(s) 1018 generally represent any type or form of hardware-implemented processing unit capable of interpreting and/or executing computer-readable instructions. In one implementation, the physical processor(s) 1018 access and/or modify one or more of the components of the optical ring control system 1004. Examples of physical processors include, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, and/or any other suitable physical processor.

Additionally as shown in FIG. 10, the optical ring 100 includes the memory 1002. In one or more implementations, the memory 1002 generally represents any type or form of volatile or non-volatile storage device or medium capable of storing data and/or computer-readable instructions. In one example, the memory 1002 stores, loads, and/or maintains one or more of the components of the optical ring control system 1004. Examples of the memory 1002 include, without limitation, Random Access Memory (RAM), Read Only Memory (ROM), flash memory, Hard Disk Drives (HDDs), Solid-State Drives (SSDs), optical disk drives, caches, variations or combinations of one or more of the same, and/or any other suitable storage memory.

Moreover, as shown in FIG. 10, the optical ring 100 includes the additional items 1014. The additional items 1014 include the input gesture data 1016. In one or more implementations, the input gesture data 1016 includes measurements and determinations taken and/or made by one or more of the optical sensor manager 1006, the bioimpedance manager 1008, the IMU manager 1010, and the input gesture manager 1012. In at least one implementation, the input gesture data 1016 further includes input gestures transmitted by the input gesture manager 1012 to additional systems.

In summary, the optical ring 100-in connection with the optical ring control system 1004-detects complex, nuanced, and stateful input gestures that a wearer can make with a hand. By enabling detection of these types of input gestures, the optical ring 100 makes it possible for the wearer to interact with digital content (e.g., via an AR or VR device) in ways that are comfortable, natural, easy, and intuitive.

### Example Implementations

Example 1: An apparatus including a ring designed to curve around a finger of a wearer, wherein the ring includes an external surface and an internal surface configured to come into contact with the finger of the wearer, a miniature optical sensor mounted on the external surface of the ring and positioned to view along a length of the finger of the wearer, and one or more electrodes mounted on the internal surface of the ring, wherein the one or more electrodes serve to measure bioimpedance in the finger of the wearer.

Example 2: The apparatus of Example 1, further including an inertial measurement unit that measures movement of the ring.

Example 3: The apparatus of Examples 1 and 2, wherein the inertial measurement unit measures movement of the ring in at least nine axes.

Example 4: The apparatus of any of Examples 1-3, further including an LED mounted in association with the miniature optical sensor.

Example 5: The apparatus of any of Examples 1-4, wherein the miniature optical sensor captures images of the finger of the wearer illuminated by the LED.

Example 6: The apparatus of any of Examples 1-5, wherein the finger of the wearer is an index finger.

Example 7: The apparatus of any of Examples 1-6, wherein the miniature optical sensor captures ultra low-resolution images of a thumb finger of the wearer interacting with the index finger of the wearer.

Example 8: A method including integrating a ring designed to curve around a finger of a wearer with a miniature optical sensor, wherein the ring includes an external surface and an internal surface configured to come into contact with the finger of the wearer and the miniature optical sensor is integrated into the external surface of the ring at a position that allows the miniature optical sensor to view along a length of the finger of the wearer, and integrating one or more electrodes into the internal surface of the ring, wherein the one or more electrodes serve to measure bioimpedance in the finger of the wearer.

Example 9: The method of Example 8, further including integrating an inertial measurement unit into the ring that measures movement of the ring in at least nine axes.

Example 10: The method of Examples 8 and 9, further including integrating an LED into the ring that illuminates images captured by the miniature optical sensor.

Example 11: The method of any of Examples 8-10, further including integrating a controller that determines movement of the ring based on images captured by the miniature optical sensor and measurements made by the inertial measurement unit.

Example 12: The method of any of Examples 8-11, wherein the controller further determines an interaction of a thumb of the wearer relative to the ring based on images captured by the miniature optical sensor and bioimpedance measurements made by the one or more electrodes.

Example 13: The method of any of Examples 8-12, wherein the interaction of the thumb of the wearer relative to the ring includes a touch of the thumb with a distal end of the finger of the wearer relative to the ring.

Example 14: The method of any of Examples 8-13, wherein the interaction of the thumb of the wearer relative to the ring includes a touch of the thumb with a proximal end of the finger of the wearer relative to the ring.

Example 15: The method of any of Examples 8-14, wherein the interaction of the thumb of the wearer relative to the ring includes a touch-and-slide movement of the thumb along the finger of the wearer away from the ring.

Example 16: The method of any of Examples 8-15, wherein the interaction of the thumb of the wearer relative to the ring includes a touch-and-slide movement of the thumb along the finger of the wearer toward the ring.

Example 17: The method of any of Examples 8-16, wherein the controller detects combination movement including movement of the ring and the interaction of the thumb of the wearer relative to the ring.

Example 18: A method including receiving one or more ultra low-resolution images captured by a miniature optical sensor integrated into an external surface of a ring worn on a finger of a wearer, receiving one or more inertial measurements from an inertial measurement unit integrated into the ring, receiving one or more bioimpedance measurements captured by one or more electrodes integrated into an internal surface of the ring, and determining a combination input gesture performed by the wearer based on the one or more ultra low-resolution images, the one or more inertial measurements, and the one or more bioimpedance measurements.

Example 19: The method of Example 18, wherein the combination input gesture includes a pinch-rotate-release input gesture that mimics the wearer turning a knob.

Example 20: The method of Examples 18 and 19, wherein the combination input gesture includes a thumb-slide/wrist-flick input gesture.

As detailed above, the computing devices and systems described and/or illustrated herein broadly represent any type or form of computing device or system capable of executing computer-readable instructions, such as those contained within the modules described herein. In their most basic configuration, these computing device(s) may each include at least one memory device and at least one physical processor.

In some examples, the term "memory device" generally refers to any type or form of volatile or non-volatile storage device or medium capable of storing data and/or computer-readable instructions. In one example, a memory device may store, load, and/or maintain one or more of the modules described herein. Examples of memory devices include, without limitation, Random Access Memory (RAM), Read Only Memory (ROM), flash memory, Hard Disk Drives (HDDs), Solid-State Drives (SSDs), optical disk drives, caches, variations or combinations of one or more of the same, or any other suitable storage memory.

In some examples, the term "physical processor" generally refers to any type or form of hardware-implemented processing unit capable of interpreting and/or executing computer-readable instructions. In one example, a physical processor may access and/or modify one or more modules stored in the above-described memory device. Examples of physical processors include, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, or any other suitable physical processor.

Embodiments of the present disclosure may include or be implemented in conjunction with various types of artificial-reality systems. Artificial reality is a form of reality that has been adjusted in some manner before presentation to a user, which may include, for example, a virtual reality, an augmented reality, a mixed reality, a hybrid reality, or some combination and/or derivative thereof. Artificial-reality content may include completely computer-generated content or computer-generated content combined with captured (e.g., real-world) content. The artificial-reality content may include video, audio, haptic feedback, or some combination thereof, any of which may be presented in a single channel or in multiple channels (such as stereo video that produces a three-dimensional (3D) effect to the viewer). Additionally, in some embodiments, artificial reality may also be associated with applications, products, accessories, services, or some combination thereof, that are used to, for example, create content in an artificial reality and/or are otherwise used in (e.g., to perform activities in) an artificial reality.

Artificial-reality systems may be implemented in a variety of different form factors and configurations. Some artificial-reality systems may be designed to work without near-eye displays (NEDs). Other artificial-reality systems may include an NED that also provides visibility into the real world (such as, e.g., augmented-reality system 1100 in FIG. 11) or that visually immerses a user in an artificial reality (such as, e.g., virtual-reality system 1200 in FIG. 12). While some artificial-reality devices may be self-contained systems, other artificial-reality devices may communicate and/or coordinate with external devices to provide an artificial-reality experience to a user. Examples of such external devices include handheld controllers, mobile devices, desktop computers, devices worn by a user, devices worn by one or more other users, and/or any other suitable external system.

Turning to FIG. 11, augmented-reality system 1100 may include an eyewear device 1102 with a frame 1110 configured to hold a left display device 1115(A) and a right display device 1115(B) in front of a user's eyes. Display devices 1115(A) and 1115(B) may act together or independently to present an image or series of images to a user. While augmented-reality system 1100 includes two displays, embodiments of this disclosure may be implemented in augmented-reality systems with a single NED or more than two NEDs.

In some embodiments, augmented-reality system 1100 may include one or more sensors, such as sensor 1140. Sensor 1140 may generate measurement signals in response to motion of augmented-reality system 1100 and may be located on substantially any portion of frame 1110. Sensor 1140 may represent one or more of a variety of different sensing mechanisms, such as a position sensor, an inertial measurement unit (IMU), a depth camera assembly, a structured light emitter and/or detector, or any combination thereof. In some embodiments, augmented-reality system 1100 may or may not include sensor 1140 or may include more than one sensor. In embodiments in which sensor 1140 includes an IMU, the IMU may generate calibration data based on measurement signals from sensor 1140. Examples of sensor 1140 may include, without limitation, accelerometers, gyroscopes, magnetometers, other suitable types of sensors that detect motion, sensors used for error correction of the IMU, or some combination thereof.

In some examples, augmented-reality system 1100 may also include a microphone array with a plurality of acoustic transducers 1120(A)-1120(J), referred to collectively as acoustic transducers 1120. Acoustic transducers 1120 may represent transducers that detect air pressure variations induced by sound waves. Each acoustic transducer 1120 may be configured to detect sound and convert the detected sound into an electronic format (e.g., an analog or digital format). The microphone array in FIG. 11 may include, for example, ten acoustic transducers: 1120(A) and 1120(B), which may be designed to be placed inside a corresponding ear of the user, acoustic transducers 1120(C), 1120(D), 1120(E), 1120(F), 1120(G), and 1120(H), which may be positioned at various locations on frame 1110, and/or acoustic transducers 1120(I) and 1120(J), which may be positioned on a corresponding neckband 1105.

In some embodiments, one or more of acoustic transducers 1120(A)-(J) may be used as output transducers (e.g., speakers). For example, acoustic transducers 1120(A) and/or 1120(B) may be earbuds or any other suitable type of headphone or speaker.

The configuration of acoustic transducers 1120 of the microphone array may vary. While augmented-reality system 1100 is shown in FIG. 11 as having ten acoustic transducers 1120, the number of acoustic transducers 1120 may be greater or less than ten. In some embodiments, using higher numbers of acoustic transducers 1120 may increase the amount of audio information collected and/or the sensitivity and accuracy of the audio information. In contrast, using a lower number of acoustic transducers 1120 may decrease the computing power required by an associated controller 1150 to process the collected audio information. In addition, the position of each acoustic transducer 1120 of the microphone array may vary. For example, the position of an acoustic transducer 1120 may include a defined position on the user, a defined coordinate on frame 1110, an orientation associated with each acoustic transducer 1120, or some combination thereof.

Acoustic transducers 1120(A) and 1120(B) may be positioned on different parts of the user's ear, such as behind the pinna, behind the tragus, and/or within the auricle or fossa. Or there may be additional acoustic transducers 1120 on or surrounding the ear in addition to acoustic transducers 1120 inside the ear canal. Having an acoustic transducer 1120 positioned next to an ear canal of a user may enable the microphone array to collect information on how sounds arrive at the ear canal. By positioning at least two of acoustic transducers 1120 on either side of a user's head (e.g., as binaural microphones), augmented-reality system 1100 may simulate binaural hearing and capture a 3D stereo sound field around about a user's head. In some embodiments, acoustic transducers 1120(A) and 1120(B) may be connected to augmented-reality system 1100 via a wired connection 1130, and in other embodiments acoustic transducers 1120(A) and 1120(B) may be connected to augmented-reality system 1100 via a wireless connection (e.g., a BLUETOOTH connection). In still other embodiments, acoustic transducers 1120(A) and 1120(B) may not be used at all in conjunction with augmented-reality system 1100.

Acoustic transducers 1120 on frame 1110 may be positioned in a variety of different ways, including along the length of the temples, across the bridge, above or below display devices 1115(A) and 1115(B), or some combination thereof. Acoustic transducers 1120 may also be oriented such that the microphone array is able to detect sounds in a wide range of directions surrounding the user wearing the augmented-reality system 1100. In some embodiments, an optimization process may be performed during manufacturing of augmented-reality system 1100 to determine relative positioning of each acoustic transducer 1120 in the microphone array.

In some examples, augmented-reality system 1100 may include or be connected to an external device (e.g., a paired device), such as neckband 1105. Neckband 1105 generally represents any type or form of paired device. Thus, the following discussion of neckband 1105 may also apply to various other paired devices, such as charging cases, smart watches, smart phones, wrist bands, other wearable devices, hand-held controllers, tablet computers, laptop computers, other external compute devices, etc.

As shown, neckband 1105 may be coupled to eyewear device 1102 via one or more connectors. The connectors may be wired or wireless and may include electrical and/or non-electrical (e.g., structural) components. In some cases, eyewear device 1102 and neckband 1105 may operate independently without any wired or wireless connection between them. While FIG. 11 illustrates the components of eyewear device 1102 and neckband 1105 in example locations on eyewear device 1102 and neckband 1105, the components may be located elsewhere and/or distributed differently on eyewear device 1102 and/or neckband 1105. In some embodiments, the components of eyewear device 1102 and neckband 1105 may be located on one or more additional peripheral devices paired with eyewear device 1102, neckband 1105, or some combination thereof.

Pairing external devices, such as neckband 1105, with augmented-reality eyewear devices may enable the eyewear devices to achieve the form factor of a pair of glasses while still providing sufficient battery and computation power for expanded capabilities. Some or all of the battery power, computational resources, and/or additional features of augmented-reality system 1100 may be provided by a paired device or shared between a paired device and an eyewear device, thus reducing the weight, heat profile, and form factor of the eyewear device overall while still retaining desired functionality. For example, neckband 1105 may allow components that would otherwise be included on an eyewear device to be included in neckband 1105 since users may tolerate a heavier weight load on their shoulders than they would tolerate on their heads. Neckband 1105 may also have a larger surface area over which to diffuse and disperse heat to the ambient environment. Thus, neckband 1105 may allow for greater battery and computation capacity than might otherwise have been possible on a stand-alone eyewear device. Since weight carried in neckband 1105 may be less invasive to a user than weight carried in eyewear device 1102, a user may tolerate wearing a lighter eyewear device and carrying or wearing the paired device for greater lengths of time than a user would tolerate wearing a heavy standalone eyewear device, thereby enabling users to more fully incorporate artificial-reality environments into their day-to-day activities.

Neckband 1105 may be communicatively coupled with eyewear device 1102 and/or to other devices. These other devices may provide certain functions (e.g., tracking, localizing, depth mapping, processing, storage, etc.) to augmented-reality system 1100. In the embodiment of FIG. 11, neckband 1105 may include two acoustic transducers (e.g., 1120(I) and 1120(J)) that are part of the microphone array (or potentially form their own microphone subarray). Neckband 1105 may also include a controller 1125 and a power source 1135.

Acoustic transducers 1120(I) and 1120(J) of neckband 1105 may be configured to detect sound and convert the detected sound into an electronic format (analog or digital). In the embodiment of FIG. 11, acoustic transducers 1120(I) and 1120(J) may be positioned on neckband 1105, thereby increasing the distance between the neckband acoustic transducers 1120(I) and 1120(J) and other acoustic transducers 1120 positioned on eyewear device 1102. In some cases, increasing the distance between acoustic transducers 1120 of the microphone array may improve the accuracy of beamforming performed via the microphone array. For example, if a sound is detected by acoustic transducers 1120(C) and 1120(D) and the distance between acoustic transducers 1120(C) and 1120(D) is greater than, e.g., the distance between acoustic transducers 1120(D) and 1120(E), the determined source location of the detected sound may be more accurate than if the sound had been detected by acoustic transducers 1120(D) and 1120(E).

Controller 1125 of neckband 1105 may process information generated by the sensors on neckband 1105 and/or augmented-reality system 1100. For example, controller 1125 may process information from the microphone array that describes sounds detected by the microphone array. For each detected sound, controller 1125 may perform a direction-of-arrival (DOA) estimation to estimate a direction from which the detected sound arrived at the microphone array. As the microphone array detects sounds, controller 1125 may populate an audio data set with the information. In embodiments in which augmented-reality system 1100 includes an inertial measurement unit, controller 1125 may compute all inertial and spatial calculations from the IMU located on eyewear device 1102. A connector may convey information between augmented-reality system 1100 and neckband 1105 and between augmented-reality system 1100 and controller 1125. The information may be in the form of optical data, electrical data, wireless data, or any other transmittable data form. Moving the processing of information generated by augmented-reality system 1100 to neckband 1105 may reduce weight and heat in eyewear device 1102, making it more comfortable to the user.

Power source 1135 in neckband 1105 may provide power to eyewear device 1102 and/or to neckband 1105. Power source 1135 may include, without limitation, lithium ion batteries, lithium-polymer batteries, primary lithium batteries, alkaline batteries, or any other form of power storage. In some cases, power source 1135 may be a wired power source. Including power source 1135 on neckband 1105 instead of on eyewear device 1102 may help better distribute the weight and heat generated by power source 1135.

As noted, some artificial-reality systems may, instead of blending an artificial reality with actual reality, substantially replace one or more of a user's sensory perceptions of the real world with a virtual experience. One example of this type of system is a head-worn display system, such as virtual-reality system 1200 in FIG. 12, that mostly or completely covers a user's field of view. Virtual-reality system 1200 may include a front rigid body 1202 and a band 1204 shaped to fit around a user's head. Virtual-reality system 1200 may also include output audio transducers 1206(A) and 1206(B). Furthermore, while not shown in FIG. 12, front rigid body 1202 may include one or more electronic elements, including one or more electronic displays, one or more inertial measurement units (IMUs), one or more tracking emitters or detectors, and/or any other suitable device or system for creating an artificial-reality experience.

Artificial-reality systems may include a variety of types of visual feedback mechanisms. For example, display devices in augmented-reality system 1100 and/or virtual-reality system 1200 may include one or more liquid crystal displays (LCDs), light emitting diode (LED) displays, microLED displays, organic LED (OLED) displays, digital light project (DLP) micro-displays, liquid crystal on silicon (LCoS) micro-displays, and/or any other suitable type of display screen. These artificial-reality systems may include a single display screen for both eyes or may provide a display screen for each eye, which may allow for additional flexibility for varifocal adjustments or for correcting a user's refractive error. Some of these artificial-reality systems may also include optical subsystems having one or more lenses (e.g., concave or convex lenses, Fresnel lenses, adjustable liquid lenses, etc.) through which a user may view a display screen. These optical subsystems may serve a variety of purposes, including to collimate (e.g., make an object appear at a greater distance than its physical distance), to magnify (e.g., make an object appear larger than its actual size), and/or to relay (to, e.g., the viewer's eyes) light. These optical subsystems may be used in a non-pupil-forming architecture (such as a single lens configuration that directly collimates light but results in so-called pincushion distortion) and/or a pupil-forming architecture (such as a multi-lens configuration that produces so-called barrel distortion to nullify pincushion distortion).

In addition to or instead of using display screens, some of the artificial-reality systems described herein may include one or more projection systems. For example, display devices in augmented-reality system 1100 and/or virtual-reality system 1200 may include micro-LED projectors that project light (using, e.g., a waveguide) into display devices, such as clear combiner lenses that allow ambient light to pass through. The display devices may refract the projected light toward a user's pupil and may enable a user to simultaneously view both artificial-reality content and the real world. The display devices may accomplish this using any of a variety of different optical components, including waveguide components (e.g., holographic, planar, diffractive, polarized, and/or reflective waveguide elements), light-manipulation surfaces and elements (such as diffractive, reflective, and refractive elements and gratings), coupling elements, etc. Artificial-reality systems may also be configured with any other suitable type or form of image projection system, such as retinal projectors used in virtual retina displays.

The artificial-reality systems described herein may also include various types of computer vision components and subsystems. For example, augmented-reality system 1100 and/or virtual-reality system 1200 may include one or more optical sensors, such as two-dimensional (2D) or 3D cameras, structured light transmitters and detectors, time-of-flight depth sensors, single-beam or sweeping laser rangefinders, 3D LiDAR sensors, and/or any other suitable type or form of optical sensor. An artificial-reality system may process data from one or more of these sensors to identify a location of a user, to map the real world, to provide a user with context about real-world surroundings, and/or to perform a variety of other functions.

The artificial-reality systems described herein may also include one or more input and/or output audio transducers. Output audio transducers may include voice coil speakers, ribbon speakers, electrostatic speakers, piezoelectric speakers, bone conduction transducers, cartilage conduction transducers, tragus-vibration transducers, and/or any other suitable type or form of audio transducer. Similarly, input audio transducers may include condenser microphones, dynamic microphones, ribbon microphones, and/or any other type or form of input transducer. In some embodiments, a single transducer may be used for both audio input and audio output.

In some embodiments, the artificial-reality systems described herein may also include tactile (i.e., haptic) feedback systems, which may be incorporated into headwear, gloves, body suits, handheld controllers, environmental devices (e.g., chairs, floormats, etc.), and/or any other type of device or system. Haptic feedback systems may provide various types of cutaneous feedback, including vibration, force, traction, texture, and/or temperature. Haptic feedback systems may also provide various types of kinesthetic feedback, such as motion and compliance. Haptic feedback may be implemented using motors, piezoelectric actuators, fluidic systems, and/or a variety of other types of feedback mechanisms. Haptic feedback systems may be implemented independent of other artificial-reality devices, within other artificial-reality devices, and/or in conjunction with other artificial-reality devices.

By providing haptic sensations, audible content, and/or visual content, artificial-reality systems may create an entire virtual experience or enhance a user's real-world experience in a variety of contexts and environments. For instance, artificial-reality systems may assist or extend a user's perception, memory, or cognition within a particular environment. Some systems may enhance a user's interactions with other people in the real world or may enable more immersive interactions with other people in a virtual world. Artificial-reality systems may also be used for educational purposes (e.g., for teaching or training in schools, hospitals, government organizations, military organizations, business enterprises, etc.), entertainment purposes (e.g., for playing video games, listening to music, watching video content, etc.), and/or for accessibility purposes (e.g., as hearing aids, visual aids, etc.). The embodiments disclosed herein may enable or enhance a user's artificial-reality experience in one or more of these contexts and environments and/or in other contexts and environments.

Throughout the drawings, identical reference characters and descriptions indicate similar, but not necessarily identical, elements. While the example embodiments described herein are susceptible to various modifications and alternative forms, specific embodiments have been shown by way of example in the drawings and will be described in detail herein. However, the example embodiments described herein are not intended to be limited to the particular forms disclosed. Rather, the present disclosure covers all modifications, equivalents, and alternatives falling within this disclosure.

As noted, augmented-reality systems 1100 and 1200 may be used with a variety of other types of devices to provide a more compelling artificial-reality experience. These devices may be haptic interfaces with transducers that provide haptic feedback and/or that collect haptic information about a user's interaction with an environment. The artificial-reality systems disclosed herein may include various types of haptic interfaces that detect or convey various types of haptic information, including tactile feedback (e.g., feedback that a user detects via nerves in the skin, which may also be referred to as cutaneous feedback) and/or kinesthetic feedback (e.g., feedback that a user detects via receptors located in muscles, joints, and/or tendons).

Haptic feedback may be provided by interfaces positioned within a user's environment (e.g., chairs, tables, floors, etc.) and/or interfaces on articles that may be worn or carried by a user (e.g., gloves, wristbands, etc.). As an example, FIG. 13 illustrates a vibrotactile system 1300 in the form of a wearable glove (haptic device 1310) and wristband (haptic device 1320). Haptic device 1310 and haptic device 1320 are shown as examples of wearable devices that include a flexible, wearable textile material 1330 that is shaped and configured for positioning against a user's hand and wrist, respectively. This disclosure also includes vibrotactile systems that may be shaped and configured for positioning against other human body parts, such as a finger, an arm, a head, a torso, a foot, or a leg. By way of example and not limitation, vibrotactile systems according to various embodiments of the present disclosure may also be in the form of a glove, a headband, an armband, a sleeve, a head covering, a sock, a shirt, or pants, among other possibilities. In some examples, the term "textile" may include any flexible, wearable material, including woven fabric, non-woven fabric, leather, cloth, a flexible polymer material, composite materials, etc.

One or more vibrotactile devices 1340 may be positioned at least partially within one or more corresponding pockets formed in textile material 1330 of vibrotactile system 1300. Vibrotactile devices 1340 may be positioned in locations to provide a vibrating sensation (e.g., haptic feedback) to a user of vibrotactile system 1300. For example, vibrotactile devices 1340 may be positioned against the user's finger(s), thumb, or wrist, as shown in FIG. 13. Vibrotactile devices 1340 may, in some examples, be sufficiently flexible to conform to or bend with the user's corresponding body part(s).

A power source 1350 (e.g., a battery) for applying a voltage to the vibrotactile devices 1340 for activation thereof may be electrically coupled to vibrotactile devices 1340, such as via conductive wiring 1352. In some examples, each of vibrotactile devices 1340 may be independently electrically coupled to power source 1350 for individual activation. In some embodiments, a processor 1360 may be operatively coupled to power source 1350 and configured (e.g., programmed) to control activation of vibrotactile devices 1340.

Vibrotactile system 1300 may be implemented in a variety of ways. In some examples, vibrotactile system 1300 may be a standalone system with integral subsystems and components for operation independent of other devices and systems. As another example, vibrotactile system 1300 may be configured for interaction with another device or system 1370. For example, vibrotactile system 1300 may, in some examples, include a communications interface 1380 for receiving and/or sending signals to the other device or system 1370. The other device or system 1370 may be a mobile device, a gaming console, an artificial-reality (e.g., virtual-reality, augmented-reality, mixed-reality) device, a personal computer, a tablet computer, a network device (e.g., a modem, a router, etc.), a handheld controller, etc. Communications interface 1380 may enable communications between vibrotactile system 1300 and the other device or system 1370 via a wireless (e.g., Wi-Fi, BLUETOOTH, cellular, radio, etc.) link or a wired link. If present, communications interface 1380 may be in communication with processor 1360, such as to provide a signal to processor 1360 to activate or deactivate one or more of the vibrotactile devices 1340.

Vibrotactile system 1300 may optionally include other subsystems and components, such as touch-sensitive pads 1390, pressure sensors, motion sensors, position sensors, lighting elements, and/or user interface elements (e.g., an on/off button, a vibration control element, etc.). During use, vibrotactile devices 1340 may be configured to be activated for a variety of different reasons, such as in response to the user's interaction with user interface elements, a signal from the motion or position sensors, a signal from the touch-sensitive pads 1390, a signal from the pressure sensors, a signal from the other device or system 1370, etc.

Although power source 1350, processor 1360, and communications interface 1380 are illustrated in FIG. 13 as being positioned in haptic device 1320, the present disclosure is not so limited. For example, one or more of power source 1350, processor 1360, or communications interface 1380 may be positioned within haptic device 1310 or within another wearable textile.

Haptic wearables, such as those shown in and described in connection with FIG. 13, may be implemented in a variety of types of artificial-reality systems and environments. FIG.14 shows an example artificial-reality environment 1400 including one head-mounted virtual-reality display and two haptic devices (i.e., gloves), and in other embodiments any number and/or combination of these components and other components may be included in an artificial-reality system. For example, in some embodiments there may be multiple head-mounted displays each having an associated haptic device, with each head-mounted display and each haptic device communicating with the same console, portable computing device, or other computing system.

Head-mounted display 1402 generally represents any type or form of virtual-reality system, such as virtual-reality system 1200 in FIG. 12. Haptic device 1404 generally represents any type or form of wearable device, worn by a user of an artificial-reality system, which provides haptic feedback to the user to give the user the perception that he or she is physically engaging with a virtual object. In some embodiments, haptic device 1404 may provide haptic feedback by applying vibration, motion, and/or force to the user. For example, haptic device 1404 may limit or augment a user's movement. To give a specific example, haptic device 1404 may limit a user's hand from moving forward so that the user has the perception that his or her hand has come in physical contact with a virtual wall. In this specific example, one or more actuators within the haptic device may achieve the physical-movement restriction by pumping fluid into an inflatable bladder of the haptic device. In some examples, a user may also use haptic device 1404 to send action requests to a console. Examples of action requests include, without limitation, requests to start an application and/or end the application and/or requests to perform a particular action within the application.

While haptic interfaces may be used with virtual-reality systems, as shown in FIG. 14, haptic interfaces may also be used with augmented-reality systems, as shown in FIG. 15. FIG. 15 is a perspective view of a user 1510 interacting with an augmented-reality system 1500. In this example, user 1510 may wear a pair of augmented-reality glasses 1520 that may have one or more displays 1522 and that are paired with a haptic device 1530. In this example, haptic device 1530 may be a wristband that includes a plurality of band elements 1532 and a tensioning mechanism 1534 that connects band elements 1532 to one another.

One or more of band elements 1532 may include any type or form of actuator suitable for providing haptic feedback. For example, one or more of band elements 1532 may be configured to provide one or more of various types of cutaneous feedback, including vibration, force, traction, texture, and/or temperature. To provide such feedback, band elements 1532 may include one or more of various types of actuators. In one example, each of band elements 1532 may include a vibrotactor (e.g., a vibrotactile actuator) configured to vibrate in unison or independently to provide one or more of various types of haptic sensations to a user. Alternatively, only a single band element or a subset of band elements may include vibrotactors.

Haptic devices 1310,1320,1404, and 1530 may include any suitable number and/or type of haptic transducer, sensor, and/or feedback mechanism. For example, haptic devices 1310, 1320, 1404, and 1530 may include one or more mechanical transducers, piezoelectric transducers, and/or fluidic transducers. Haptic devices 1310, 1320, 1404, and 1530 may also include various combinations of different types and forms of transducers that work together or independently to enhance a user's artificial-reality experience. In one example, each of band elements 1532 of haptic device 1530 may include a vibrotactor (e.g., a vibrotactile actuator) configured to vibrate in unison or independently to provide one or more of various types of haptic sensations to a user.

The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed. The various example methods described and/or illustrated herein may also omit one or more of the steps described or illustrated herein or include additional steps in addition to those disclosed.

The preceding description has been provided to enable others skilled in the art to best utilize various aspects of the example embodiments disclosed herein. This example description is not intended to be exhaustive or to be limited to any precise form disclosed. Many modifications and variations are possible without departing from the scope of the present disclosure. The embodiments disclosed herein should be considered in all respects illustrative and not restrictive. Reference should be made to any claims appended hereto and their equivalents in determining the scope of the present disclosure.

Unless otherwise noted, the terms "connected to" and "coupled to" (and their derivatives), as used in the specification and/or claims, are to be construed as permitting both direct and indirect (i.e., via other elements or components) connection. In addition, the terms "a" or "an," as used in the specification and/or claims, are to be construed as meaning "at least one of." Finally, for ease of use, the terms "including" and "having" (and their derivatives), as used in the specification and/or claims, are interchangeable with and have the same meaning as the word "comprising."

## Claims

1. An apparatus comprising:
a ring designed to curve around a finger of a wearer, wherein the ring comprises an external surface and an internal surface configured to come into contact with the finger of the wearer;
a miniature optical sensor mounted on the external surface of the ring and positioned to view along a length of the finger of the wearer; and
one or more electrodes mounted on the internal surface of the ring, wherein the one or more electrodes serve to measure bioimpedance in the finger of the wearer.

2. The apparatus of claim 1, further comprising an inertial measurement unit that measures movement of the ring.

3. The apparatus of claim 2, wherein the inertial measurement unit measures movement of the ring in at least nine axes.

4. The apparatus of any preceding claim, further comprising an LED mounted in association with the miniature optical sensor.

5. The apparatus of claim 4, wherein the miniature optical sensor captures images of the finger of the wearer illuminated by the LED.

6. The apparatus of claim 5, wherein the finger of the wearer is an index finger.

7. The apparatus of claim 6, wherein the miniature optical sensor captures ultra low-resolution images of a thumb finger of the wearer interacting with the index finger of the wearer.

8. A method comprising:
integrating a ring designed to curve around a finger of a wearer with a miniature optical sensor,
wherein the ring comprises an external surface and an internal surface configured to come into contact with the finger of the wearer and
the miniature optical sensor is integrated into the external surface of the ring at a position that allows the miniature optical sensor to view along a length of the finger of the wearer; and
integrating one or more electrodes into the internal surface of the ring, wherein the one or more electrodes serve to measure bioimpedance in the finger of the wearer.

9. The method of claim 8, further comprising integrating an inertial measurement unit into the ring that measures movement of the ring in at least nine axes.

10. The method of claim 8 or claim 9, further comprising integrating an LED into the ring that illuminates images captured by the miniature optical sensor.

11. The method of claim 10, further comprising integrating a controller that determines movement of the ring based on images captured by the miniature optical sensor and measurements made by the inertial measurement unit,
and further optionally wherein the controller further determines an interaction of a thumb of the wearer relative to the ring based on images captured by the miniature optical sensor and bioimpedance measurements made by the one or more electrodes.

12. The method of claim 11, wherein the interaction of the thumb of the wearer relative to the ring comprises:
a touch of the thumb with a distal end of the finger of the wearer relative to the ring,
a touch of the thumb with a proximal end of the finger of the wearer relative to the ring,
a touch-and-slide movement of the thumb along the finger of the wearer away from the ring, or
a touch-and-slide movement of the thumb along the finger of the wearer toward the ring.

13. The method of claim 11 or claim 12, wherein the controller detects combination movement including movement of the ring and the interaction of the thumb of the wearer relative to the ring.

14. A method comprising:
receiving one or more ultra low-resolution images captured by a miniature optical sensor integrated into an external surface of a ring worn on a finger of a wearer;
receiving one or more inertial measurements from an inertial measurement unit integrated into the ring;
receiving one or more bioimpedance measurements captured by one or more electrodes integrated into an internal surface of the ring; and
determining a combination input gesture performed by the wearer based on the one or more ultra low-resolution images, the one or more inertial measurements, and the one or more bioimpedance measurements.

15. The method of claim 14, wherein the combination input gesture comprises:
a pinch-rotate-release input gesture that mimics the wearer turning a knob, or
a thumb-slide/wrist-flick input gesture.
